Europäisches Patentamt

European Patent Office

Office européen des brevets

(19)

(11) Veröffentlichungsnummer: **0 303 174 B1**

(12) # EUROPÄISCHE PATENTSCHRIFT

(45) Veröffentlichungstag der Patentschrift: **19.11.92**

(21) Anmeldenummer: **88112644.5**

(22) Anmeldetag: **03.08.88**

(51) Int. Cl.⁵: **A61F  9/00**

㉟ ⑤④ **Vorrichtung zur chirurgischen Korrektur von Fehlsichtigkeiten eines oder beider Augen von Lebewesen.**

(30) Priorität: **10.08.87 CH 3056/87**

(43) Veröffentlichungstag der Anmeldung:
**15.02.89 Patentblatt  89/07**

(45) Bekanntmachung des Hinweises auf die Patenterteilung:
**19.11.92 Patentblatt  92/47**

(84) Benannte Vertragsstaaten:
**AT BE CH DE ES FR GB GR IT LI LU NL SE**

(56) Entgegenhaltungen:
**EP-A- 0 218 427**
**US-A- 4 173 980**

(73) Patentinhaber: **GRIESHABER & CO. AG SCHAFFHAUSEN**
**Winkelriedstrasse 52**
**CH-8203 Schaffhausen(CH)**

(72) Erfinder: **Grieshaber, Hans Rudolf**
**Winkelriedstrasse 52**
**CH-8203 Schaffhausen(CH)**

(74) Vertreter: **EGLI-EUROPEAN PATENT ATTORNEYS**
**Horneggstrasse 4**
**CH-8008 Zürich(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung bezieht sich auf eine Vorrichtung zur Korrektur einer im optischen System eines Auges von der Hornhautkrümmung abhängigen Fehlsichtigkeit, mit einem im optischen Zentrum durch Vakuum am Auge gehaltenen Basiskörper und einem rotierend angetriebenen Schleif- oder Polierstempel, welcher auf der der Hornhaut-Oberfläche des Auges zugewandten Seite mit einer Schleifläche versehen ist.

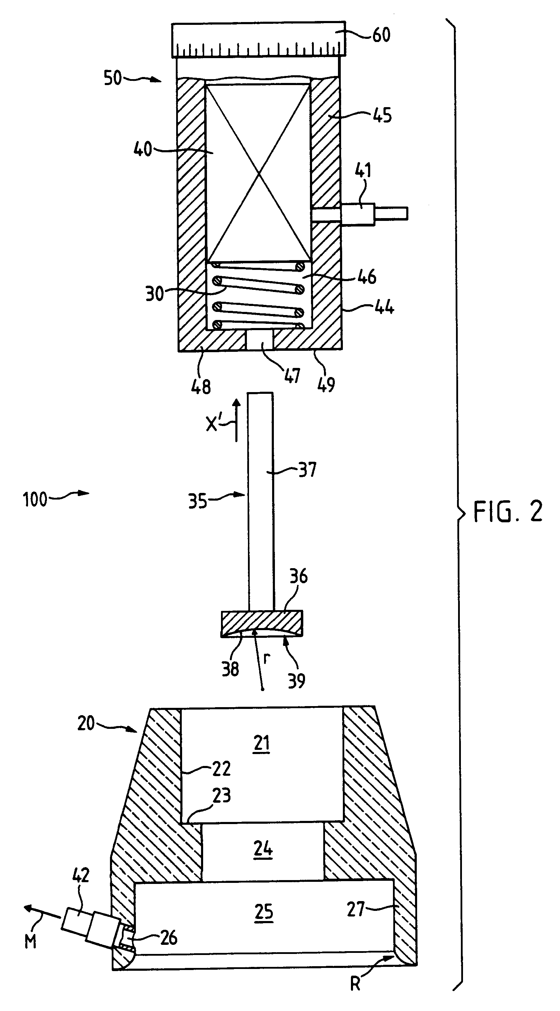

Aus der US-A 4 173 980 ist eine Vorrichtung zur Korrektur von Fehlsichtigkeiten bekannt. Die im wesentlichen als Stativ ausgebildete Vorrichtung umfasst eine an einem auslegerartig ausgebildeten Ständer angeordnete Schleifvorrichtung, welche mittels einer Verstellvorrichtung (Fig.2) in vertikaler Richtung relativ zu einer Kopfauflage beziehungsweise relativ zu dem zu behandelnden Auge verstellbar ist. Bei dieser Vorrichtung wird in einer ersten Phase das Auge mit einem ringartigen Halteelement in seiner Lage positioniert, und anschliessend wird mittels eines um eine vertikale Achse rotierend angetriebenen Schleiforgans mit konkav ausgebildeter Schleiffläche oder mittels einer um eine etwa horizontale Achse rotierend angetriebenen Schleifrolle die Hornhaut-Oberfläche geschliffen. Der konstruktive Aufbau der Vorrichtung und die erforderliche Fixierung zur Erreichtung der einzelnen Funktionen sind entsprechend kompliziert und aufwendig.

Allgemein geht man bei der objektiven Refraktionsbestimmung (Brechungszustand eines oder beider Augen) von der Kenntnis aus, dass im optischen System bei einem normalsichtigen (emmetropen) Auge die gekrümmte Hornhaut und die natürliche Linse im wesentlichen die parallel einfallenden Lichtstrahlen brechen, so dass ihr Brennpunkt (Schnittpunkt) in der Netzhautebene liegt. Besteht jedoch ein Missverhältnis zwischen der Brechkraft und der Achsenlänge des optischen Systems, so liegt eine Fehlsichtigkeit (Ametropie) vor, wobei bei einem kurzsichtigen (myopen) Auge der Brennpunkt vor der Netzhaut und bei einem weitsichtigen (hyperopen) Auge der Brennpunkt hinter der Netzhaut liegt. Bilden die einfallenden Lichtstrahlen keinen gemeinsamen Brennpunkt (Astigmatismus), so ist die Krümmung der Hornhaut in verschiedenen Richtungen unterschiedlich gross, wodurch eine sogenannte Verzerrung entsteht.

Die primären Korrekturmittel für derartige Fehlsichtigkeiten sind im wesentlichen Brillen, Kontaktlinsen oder dergleichen. Bei extremeren Fehlsichtigkeiten ist es weiterhin bekannt, eine Korrektur durch operative Eingriffe vorzunehmen. Hierbei wird beispielsweise durch Keratoplastik, Keratophakie, Epikeratophakie oder Keratomileusis eine Veränderung der Hornhautkrümmung und dadurch eine Verlegung des Brennpunktes auf die Netzhaut erreicht. Nach einem weiteren operativen Korrekturverfahren für geringere Fehlsichtigkeiten werden mehrere radiale Schnitte in die Peripherie der Hornhaut gelegt, wodurch die Hornhaut an Krümmungsradius verliert und somit eine geringere Brechkraft bekommt.

Diese unter dem Begriff "Radiäre Keratotomie" bekannte Operationsmethode ist ausschliesslich auf das myope Auge begrenzt.

Bei den vorstehend beschriebenen, an sich traumatischen Korrekturverfahren wird die Hornhaut (Cornea) aufgrund der Tiefe der durchzuführenden radiären Einschnitte einerseits erheblich geschwächt, andererseits ist eine graduelle Rückentwicklung zu den präoperativen Krümmungsradien der Hornhaut und somit eine Wiederkehr des myopischen, hyperopischen oder astigmatischen Zustands des jeweiligen Auges nicht auszuschliessen.

Der Erfindung liegt somit die Aufgabe zugrunde, eine zur Korrektur der von der Hornhautkrümmung eines Auges abhängigen Fehlsichtigkeit ausgebildete Vorrichtung dahingehend zu verbessern, dass der rotierend angetriebene Schleif- oder Polierstempel mit exakt auf die Fehlsichtigkeit hinsichtlich des Korrekturradius sowie der Schleiftiefe abgestimmten Referenz-Werten der Hornhaut-Oberfläche zustellbar ist.

Die erfindungsgemässe Vorrichtung ist dadurch gekennzeichnet, dass der Basiskörper zur selbstzentrierenden Aufnahme eines mit dem Schleif- oder Polierstempels wirkverbundenen Schleifkopfes mit in axialer Richtung des optischen Zentrums orientierten und den Basiskörper durchdringenden Ausnehmungen versehen ist, wobei die zylindrische Seitenwand der ersten, zur Aufnahme des Schleifkopfes ausgebildeten Ausnehmung als eine in radialer Richtung zum Zentrum des optischen Systems orientierte Referenz und eine zwischen der ersten Ausnehmung und der zweiten Ausnehmung vorgesehene, kreisringförmig ausgebildete Auflagefläche als eine in axialer Richtung zur Hornhaut-Oberfläche des Auges orientierte Referenz ausgebildet ist.

Mit der erfindungsgemässen Vorrichtung können sämtliche, im wesentlichen durch die Hornhautkrümmung verursachte Fehlsichtigkeiten an eine im Bereich der optischen Zone der Hornhaut liegende Hornhautkrümmung angepasst und korrigiert werden.

Weitere Merkmale der Erfindung ergeben sich aus der folgenden Beschreibung in Verbindung mit der Zeichnung und den weiteren Patentansprüchen.

Die Erfindung wird nachstehend in Verbindung mit der Zeichnung beschrieben. Es zeigt:

Fig.1 in schematischer Darstellung eine Augenhornhaut-Schleifvorrichtung, und

Fig.2 die als Sprengzeichnung dargestellte Schleifvorrichtung gemäss Fig.1.

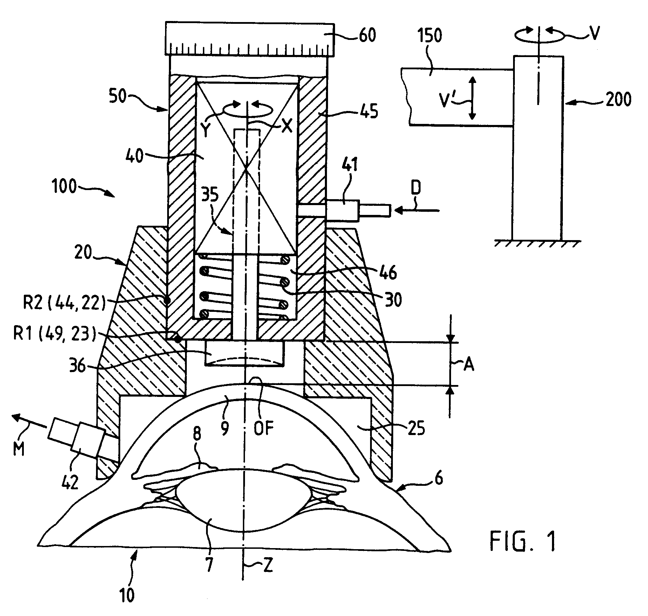

Fig.1 zeigt als Ausführungsbeispiel eine in grösserem Massstab und in schematischer Schnittansicht dargestellte Augenhornhaut-Schleifvorrichtung, welche in ihrer Gesamtheit mit 100 bezeichnet und zur korrigierenden Krümmungsänderung der Augenhornhaut eines Lebewesens ausgebildet ist. Die Schleifvorrichtung 100 umfasst im wesentlichen einen mit 50 bezeichneten Schleifkopf (GRINDER) sowie einen mit 20 bezeichneten Basiskörper, welcher einerseits zur Aufnahme des Schleifkopfes 50 und andererseits zur strengen, sterilen Fixierung an dem in Fig.1 schematisch dargestellten und mit 10 bezeichneten Auge ausgebildet ist.

Bei dem nur teilweise und zur besseren Erläuterung ebenfalls in grösserem Massstab dargestellten Auge 10 ist mit 9 die als optisches Fenster des Auges wirkende Hornhaut (Cornea), mit 8 die als Blende des Auges wirkende Regenbogenhaut (Iris), mit 7 die als dioptrisches Organ wirkende Linse und mit 6 im wesentlichen die Bindehaut (Conjunctiva) bezeichnet. Die in Fig.1 als nur eine Schicht dargestellte Hornhaut 9 (Cornea) ist realistisch und im Querschnitt betrachtet ein nicht näher dargestelltes 5-schichtiges Gewebe, welches

- aus relativ dünnen oberflächlichen Hornhautepithel mit Basalmembran,
- aus darunter liegenden, relativ dünnen Bowmanschen-Membran,
- aus den grössten Anteil der Hornhaut darstellenden Hornhautstroma,
- aus Descemetschen-Membran, und
- aus daraufliegenden, relativ dünnen Endothelschicht besteht,

wobei die Descemetsche-Membran und die Endothelschicht zur Augen-Vorderkammer hin gesehen sich an das Hornhautstroma anschliessen. Das 5-schichtige Hornhaut-Gewebe ist wie bekannt von transparenter Beschaffenheit und hat eine gewisse Elastizität.

Der Schleifkopf 50 umfasst im wesentlichen ein teilweise im Schnitt dargestelltes Gehäuse 45, in welchem ein Antriebsorgan 40 , ein von dem Antriebsorgan 40 um eine senkrechte Achse X in Pfeilrichtung Y rotierend angetriebener Schleif-oder Polierstempel 35 sowie eine schematisch dargestellte Rückstellfeder 30 angeordnet ist. Zur Aufnahme der Teile 30,35 und 40 ist das Gehäuse 45 mit einer entsprechend ausgebildeten Ausnehmung 46 versehen. Am oberen Ende des Gehäuses 45 ist ein Einstellorgan, vorzugsweise eine Mikrometerschraube 60, dessen Funktion später noch ausführlich beschrieben wird, oder dergleichen angeordnet und mit nicht dargestellten Mitteln befestigt.

An dieser Stelle sei darauf hingewiesen, dass als Antriebsorgan 40 für den Schleif-oder Polierstempel 35 beispielsweise eine hochtourige Luftturbine, ein Gleichstrommotor, ein Schrittmotor oder ein externer Antrieb (nicht dargestellt) vorgesehen sein kann, wobei bei Verwendung eines externen Antriebes dieser über eine zusätzliche Welle mit dem Schleif-oder Polierstempel 35 in Wirkverbindung steht. Im dargestellten Ausführungsbeispiel ist als Antriebsorgan eine an sich bekannte, schematisch dargestellte Turbine mit etwa 250.000 Umdrehungen/Minute vorgesehen, welche über einen am Gehäuse 45 angeordneten Stutzen 41 mit Druckluft D versorgt wird.

In Fig.2 ist die Augenhornhaut-Schleifvorrichtung 100 als Sprengzeichnung dargestellt und man erkennt das Gehäuse 45 des Schleifkopfes, den Schleif-oder Polierstempel 35 sowie den Basiskörper 20, welche Teile 45,35 und 20 nachstehend im Einzelnen beschrieben werden:

Wie bereits erwähnt, ist das Gehäuse 45 zur Aufnahme der Turbine 40 mit der Ausnehmung 46 versehen und im oberen Bereich zur Aufnahme der Mikrometerschraube 60 ausgebildet. Im unteren Bereich weist das Gehäuse 45 einen von einer Bohrung 47 durchdrungenen Boden 48 auf, welcher mit seiner äusseren Fläche 49 als Referenzfläche zu einer entsprechenden Referenzfläche 23 des Basiskörpers 20 dient.

Der Schleif-oder Polierstempel 35 hat einen Wellenzapfen 37, an welchem an dem einen Ende eine tellerartig ausgebildete Stempelscheibe 36 angeordnet, vorzugsweise angeformt ist. Die im Schnitt dargestellte Stempelscheibe 36 ist an der Unterseite 39 mit einer im wesentlichen konkav ausgebildeten Fläche 38 versehen. Die konkave Fläche 38, deren spezielle Formgebung und Grösse später noch ausführlich beschrieben wird, ist mit einer feinkörnig kristallisierten, vorzugsweise mit Diamantstaub belegten Oberflächen-Struktur versehen, wobei die Diamantkristallgrösse so gewählt ist, dass für das erfindungsgemässe Verfahren eine entsprechende Schleif-oder Polierwirkung erreicht wird. Der in Pfeilrichtung X' (Fig.2) in das Gehäuse 45 sowie in die Turbine 40 einführbare und vorzugsweise an der Gehäusebohrung 47 zentrierte Schleif-oder Polierstempel 35 ist in zusammengebautem Zustand (Fig.1), über seinen Wellenzapfen 37 mit der Turbine in nicht näher dargestellter Weise wirkverbunden und wird von dieser für den Schleif-oder Poliervorgang in Pfeilrichtung Y angetrieben. Die drehfeste Wirkverbindung des Schleif-oder Polierstempels 35 in der Turbine 40 ist so ausgebildet, dass ein rasches Auswechseln ohne weitere Hilfsmittel gewährleistet ist.

Der Basiskörper 20 hat eine erste Ausnehmung 21, eine zweite Ausnehmung 24 sowie eine dritte Ausnehmung 25. Die dritte Ausnehmung 25 bildet nach Fixierung des Basiskörpers 20 am Auge 10 einen sogenannten Vakuumraum, welcher über eine Öffnung 26 mit einem Absaugstutzen 42 in Verbindung steht. Für eine optimal abschliessende Anlage der Wand 27 des Vakuumraumes 25 im Bereich der mit 6 bezeichneten Bindehaut ist die Wand 27 an der Innenseite mit einem Radius R versehen. Die erste Ausnehmung 21 ist zur Aufnahme des gesamten Schleifkopfes 50 gebildet, dessen unterer Gehäusebereich 44 im zusammengebauten Zustand (Fig.1) an der Seitenwand 22 zentriert und geführt ist, während die untere Gehäusefläche 49 an der ringförmigen Fläche 23 des Basiskörpers 20 aufliegt. Der Basiskörper 20 besteht aus gut sterilisierbarem Werkstoff, vorzugsweise aus transparentem Werkstoff, beispielsweise aus transparentem Plexiglas oder dergleichen. In zusammengebautem und am Auge 10 fixierten Zustand (Fig.1) der Schleifvorrichtung 100, bilden die beiden als Auflage dienenden Flächen 49,23 der Teile 20,45 eine Referenz R1 in axialer Richtung zu der Hornhaut-Oberfläche OF und die beiden zylindrischen Wände 44,22 der Teile 20,45 eine Referenz R2 in radialer Richtung zum optischen Zentrum Z der Hornhaut 9. Das mit Z bezeichnete optische Zentrum entspricht der ausschliesslich nur in einem schematisch dargestelltem Auge genau definierbaren, vom Hornhautscheitel bis zum hinteren, nicht dargestellten Augenpol verlaufenden optischen Achse (Fig.2).

Die Arbeitsweise der Vorrichtung zur Korrektur der von der Hornhautkrümmung abhängigen Fehlsichtigkeit wird nachstehend anhand der im einzelnen aufgeführten, wesentlichen Schritte beschrieben:

Schritt 1 - Feststellen der Refraktion (Brechungszustand) des einzelnen fehlsichtigen Auges mittels einer an sich bekannten Refraktionsmethode:

Schritt 2 - Bestimmen der Hornhautkrümmung mittels entsprechend auseebildetem Ophthalmometer, bei welchem vorzugsweise der jeweilige, von verschiedenen Grössen abhängige Krümmungsradius der Hornhaut direkt ablesbar ist;

Schritt 3 - Berechnung des erforderlichen Korrekturwertes, d.h., des Korrekturradius unter Berücksichtigung der anhand von Schritt 2 ermittelten Hornhautkrümmung;

Schritt 4 - Berechnung der Schleiftiefe unter Berücksichtigung der im Schritt 2 und 3 ermittelten Parameter, d.h., unter Berücksichtigung der präoperativen Hornhautkrümmung und des im Schritt 3 berechneten Korrekturradius;

Schritt 5 - Bestimmen des optischen Zentrums Z der jeweiligen Augen-Hornhaut 9 und exakt zentrisches Aufsetzen und Befestigen des Basiskörpers 20 am Auge 10;

Schritt 6 - Bestimmen des mit A bezeichneten Abstandes von der Referenzfläche R1 bis zur Hornhaut-Oberfläche OF;

Schritt 7 - Einstellen der Mikrometer-Schraube 60 auf die anhand von Schritt 4 berechnete Schleiftiefe zuzüglich (plus) dem anhand von Schritt 6 ermittelten Abstand A (Offset);

Schritt 8 - Auswahl des entsprechend ausgebildeten (Korrekturradius gemäss Schritt 3) Schleif- oder Polierstempels 35, Einsetzen in den Schleifkopf 50 und Sterilisation des gesamten Schleifkopfes.

Im Anschluss an die vorstehenden, im wesentlichen einer Vorbereitungsphase entsprechenden Schritte 1 bis 8 erfolgt nunmehr unter absolut sterilen Voraussetzungen der eigentliche operative Eingriff an der Hornhaut-Oberfläche OF derart, dass der Schleifkopf 50 (GRINDER) in die Ausnehmung 21 des Basiskörpers 20 eingesetzt und anschliessend der Schleif-oder Polierstempel 35 mit der entsprechenden Tourenzahl angetrieben sowie der anhand der Schritte 2, 3 und 4 ermittelten Parameter für den Schleif-oder Poliervorgang der Hornhaut-Oberfläche OF zugeführt wird. Die am Schleifkopf 50 vorgesehene Mikrometerschraube 60 gewährleistet hierbei in nicht näher dargestellter Weise eine hochpräzise und durch einen Anschlag (nicht dargestellt) begrenzte Zustellbewegung des Schleif- oder Polierstempels 35. Unter Berücksichtigung der ermittelten Parameter wird beim Abschleifen oder Abpolieren, d.h., beim eigentlichen operativen Eingriff, die erste relativ dünne Hornhautepithel-Schicht einschliesslich der Bowmanschen-Membran sowie ein Teil des Hornhautstromas abgetragen, wobei das hierbei freigelegte Hornhautstroma nach einer gewissen Zeit von Epithelzellen wieder überwachsen wird.

Bei der in Fig.1 dargestellten Schleifvorrichtung 100 wird der Basiskörper 20 mit dem montierten Schleifkopf 50 am Auge 10 mit nicht näher dargestellten Mitteln gehalten,d.h., dass die einzelnen Elemente relativ klein mit geringem Eigengewicht auszubilden sind ohne jedoch die erforderliche Stabilität zu vernachlässigen.

Die lösbare Befestigung des die Hornhaut (Cornea) zirkulär umgreifenden Basiskörpers 20 am Auge 10 selbst oder im äusseren Bereich des Auges kann bespielsweise durch Nähen, Klammern oder dergleichen erfolgen. Im dargestellten Beispiel wird der Basiskörper 20 durch Erzeugung von Vakuum im Raum 25 oberflächig an der Bindehaut 6 (Conjunctiva) des Auges 10 gehalten, hierzu wird mit nicht dargestellten Mitteln über den Ansaugstutzen 42 im Raum 25 ein Vakuum erzeugt und während des Eingriffs weitgehend konstant gehalten.

Bei dem als Ausführungsbeispiel vorstehend beschriebenen Schleifkopf 50 ist der Schleif- oder Polierstempel 35 als sogenannte steigende Spindel ausgebildet, welche in montiertem Zustand die Zustellbewegung zur Hornhaut-Oberfläche OF hin sowie die Rotationsbewegung ausführt. Bei einem in Fig.1 schematisch angedeuteten Beispiel besteht jedoch auch die Möglichkeit, den Schleifkopf 50 an einem teilweise dargestellten und in Pfeilrichtung V wegschwenkbaren sowie in Pfeilrichtung V′ höhenverstellbaren Auslegerarm 150 eines stativartigen Ständers 200 oder dergleichen lösbar anzuordnen. Bei dieser Variante wird der Schleifkopf 50 zusammen mit dem Schleif- oder Polierstempel 35 der Hornhaut-Oberfläche OF gut reproduzierbar zugestellt, wobei der Schleif- oder Polierstempel 35 lediglich die Rotationsbewegung ausführt.

Bei der vorstehend beschriebenen Hochgeschwindigkeits-Schleiftechnik kann während des chirurgischen Eingriffs eine Kühlung erforderlich sein. Ein hierzu geeignetes Medium ist zum Beispiel eine visco-elastische Substanz, welche über ein nicht dargestelltes Zirkulations-Kanalsystem dem Schleif-oder Polierstempel 35 im Bereich der Stempelscheibe 36 zugeführt und über den Stutzen 42 in Pfeilrichtung M abgesogen werden kann.

Wie bereits erwähnt, ist der auswechselbare Schleif- oder Polierstempel 35 an der Unterseite der Stempelscheibe 36 mit einer bei der Rotation an der jeweiligen Hornhaut-Oberfläche OF als Schleiffläche wirkenden, konkaven Fläche 38 versehen. Aufgrund der Tatsache, dass der Hornhaut-Krümmungsradius von einem zum anderen Auge naturgemäss differenziert, so sind auch für die erfindungsgemässe Korrektur der Fehlsichtigkeit des einzelnen Auges die mit r bezeichneten Krümmungsradien der konkaven Fläche 38 entsprechend auszubilden. Der jeweilige Krümmungsradius r liegt im Grössenbereich von 5 bis 15 mm, vorzugsweise im Bereich von 6,5 bis 12 mm mit einer im Bereich von 1/10 mm liegenden Abstufung. Die Wahl des jeweils einzusetzenden oder zu verwendenden Schleif- oder Polierstempels 35 ist abhängig von den anhand der Schritte 2, 3 oder 4 (Hornhautkrümmung, Korrekturwert, Schleiftiefe) ermittelten oder berechneten Werten.

Ein wesentlicher Faktor bei der Wahl des zu verwendenden Schleif- oder Polierstempels 35 wird darin gesehen, dass das Verhältnis Schleiftiefe/Schleifdurchmesser so gewählt wird, dass nach dem Abschleifen oder Abpolieren die freigelegte und anschliessend von den Epithelzellen wieder überwachsene Hornhautoberfläche OF eine für die gewünschte, optimale Sehfunktion des einzelnen Auges erforderliche glatte Oberfläche aufweist.

Aufgrund des erforderlichen umfassenden Sortiments an auswechselbaren Schleif- oder Polierstempeln 35 besteht die Möglichkeit, diesen zweiteilig, bestehend aus Wellenzapfen 37 und Stempelscheibe 36, auszubilden und lediglich die mit der entsprechend konkaven Fläche 38 versehene Stempelscheibe 36 auszuwechseln und mit nicht näher dargestellten Mitteln zu befestigen.

## Patentansprüche

1. Vorrichtung zur Korrektur einer im optischen System eines Auges von der Hornhautkrümmung abhängigen Fehlsichtigkeit, mit einem im optischen Zentrum (Z) durch Vakuum am Auge (10) gehaltenen Basiskörper (20) und einem rotierend angetriebenen Schleif- oder Polierstempel (35), welcher auf der der Hornhaut-Oberfläche (OF) des Auges (10) zugewandten Seite mit einer Schleiffläche (38) versehen ist, dadurch gekennzeichnet, dass der Basiskörper (20) zur selbstzentrierenden Aufnahme eines mit dem Schleif- oder Polierstempel (35) wirkverbundenen Schleifkopfes (50) mit in axialer Richtung des optischen Zentrums (Z) orientierten und den Basiskörper (20) durchdringenden Ausnehmungen (21,24,25) versehen ist, wobei die zylindrische Seitenwand (22) der ersten, zur Aufnahme des Schleifkopfes (50) ausgebildeten Ausnehmung (21) als eine in radialer Richtung zum Zentrum (Z) des optischen Systems orientierte Referenz (R2) und eine zwischen der ersten Ausnehmung (21) und der zweiten Ausnehmung (24) vorgesehene, kreisringförmig ausgebildete Auflagefläche (23) als eine in axialer Richtung zur Hornhaut-Oberfläche (OF) des Auges (10) orientierte Referenz (R1) ausgebildet ist.

2. Vorrichtung nach Anspruch 1, dadurch gekennzeichnet, dass der Schleif- oder Polierstempel (35) auswechselbar in dem Schleifkopf (50) angeordnet und die konkav ausgebildete Schleiffläche (38) des jeweils eingesetzten Schleif- oder Polierstempels (35) mit einem von der Refraktion des fehlsichtigen Auges abhängigen Radius (r) versehen ist.

3. Vorrichtung nach den Ansprüchen 1 und 2, dadurch gekennzeichnet, dass der Schleifkopf (50) mit einer Mikrometerschraube (60) versehen ist, mittels welcher der Schleif- oder Polierstempel (35) mit einer Schleiftiefe, welche von der Refraktion des fehlsichtigen Auges sowie von einem zwischen der Referenzfläche (R1) und der Hornhaut-Oberfläche (OF) gebildeten Abstand (A) abhängt, dem Auge (10) zustellbar ist.

4. Vorrichtung nach Anspruch 1, dadurch gekennzeichnet, dass die konkav ausgebildete Schleiffläche (38) des Schleif- oder Polierstempels (35) mit feinkörnig kristallisierter Oberflächenstruktur oder mit Diamantstaub versehen ist.

5. Vorrichtung nach den Ansprüchen 1 und 3, dadurch gekennzeichnet, dass
   - der Schleif- oder Polierstempel (35) von einem im Schleifkopf (50) oder von einem extern angeordneten, hochtourigen Antriebsaggregat angetrieben wird, und
   - der Schleifkopf (50) an einem an sich bekannten Auslegerarm (150) eines stativartig ausgebildeten Ständers (200) angeordnet ist, und
   - der Auslegerarm (150) zusammen mit dem Schleifkopf (50) und dem Schleif- oder Polierstempel (35) in axialer Richtung des optischen Zentrums (Z) mit der von der Refraktion des fehlsichtigen Auges abhängigen Schleiftiefe dem Auge (10) zustellbar ist.

## Claims

1. Apparatus for the correction of defective sight dependent upon the curvature of the cornea in the optical system of an eye, having a base body (20) which is held at the eye (10) by means of vacuum in the optical centre (Z) and a rotary driven grinding or polishing plunger (35) which is provided with a grinding surface (38) on the side facing the upper cornea surface (OF) of the eye (10), characterised in that for the purpose of receiving in a self-centering manner a grinding head (50) which is operatively connected with the grinding or polishing plunger (35), the base body (20) is provided with recesses (21, 24, 25) which are orientated in the axial direction of the optical centre (Z) and penetrate the base body (20), with the cylindrical side wall (22) of the first recess (21), which is formed for the purpose of receiving the grinding head (50), being formed as a reference (R2) which is orientated in a radial direction relative to the centre (Z) of the optical system, and a supporting surface (23), which is provided between the first recess (21) and the second recess (24) and is formed in the shape of a circular ring, being formed as a reference (R1) which is orientated in an axial direction relative to the upper cornea surface (OF) of the eye (10).

2. Apparatus according to claim 1, characterised in that the grinding or polishing plunger (35) is arranged in the grinding head (50) such that it is capable of being exchanged and the concavely formed grinding surface (38) of the respective grinding or polishing plunger (35) used is provided with a radius (r) which is dependent upon the refraction of the eye with defective sight.

3. Apparatus according to claims 1 and 2, characterised in that the grinding head (50) is provided with a micrometer screw (60) by means of which the grinding or polishing plunger (35) can be advanced to the eye (10) with a grinding depth which is dependent upon the refraction of the eye with defective sight as well as upon a distance (A) which is established between the reference surface (R1) and the upper cornea surface (OF).

4. Apparatus according to claim 1, characterised in that the concavely formed grinding surface (38) of the grinding or polishing plunger (35) is provided with a surface structure which is crystallised in a fine-grained manner or is provided with diamond dust.

5. Apparatus according to claims 1 and 3, characterised in that
   - the grinding or polishing plunger (35) is driven by a high-speed drive unit which is arranged in the grinding head (50) or by a high-speed drive unit which is arranged externally; and
   - the grinding head (50) is arranged on a bracket arm (150), which is known per se, of a mount (200) which is formed in the manner of a stand; and
   - the bracket arm (150) can be advanced to the eye (10) together with the grinding head (50) and the grinding or polishing plunger (35) in the axial direction of the optical centre (Z) with the grinding depth which is dependent upon the refraction of the eye with defective sight.

## Revendications

1. Appareil pour la correction d'une opacité de la vue dans le système optique d'un oeil en rapport avec une modification de la cornée avec un corps de base (20) maintenu par vide sur l'oeil (10) dans le centre optique (Z) et un porte-outil (35) de ponçage ou de polissage entraîné en rotation, dont le côté tourné vers la surface de la cornée (OF) de l'oeil (10) est pourvu d'une surface de ponçage (38) caractérisé en ce que le corps de base (20) est pourvu d'évidements (21,24,25) orientés dans le sens axial du centre optique (Z) et traversant le corps de base (20) pour recevoir en la centrant automatiquement une tête de ponçage (50) en liaison active avec le porte-outil (35) de ponçage ou de polissage, la paroi latérale cylindrique 22 du premier évidement (21) formé pour recevoir la tête de ponçage 50 étant conçue comme un point de référence (R2) orientée dans le sens axial du centre optique (Z) et une surface d'appui (23) conçue en forme de couronne prévue entre la première cavité (21) et la deuxième cavité (24) étant conçue comme un point de référence (R1) dans le sens axial de la surface (OF) de la cornée de l'oeil (10).

2. Appareil selon la revendication 1, caractérisé en ce que le porte-outil de ponçage ou de polissage (35) est disposé dans la tête de ponçage (50) de manière à pouvoir être changé et que la surface de ponçage (38) concave du porte-outil de ponçage ou de polissage (35) utilisé est pourvue d'un rayon (r) dépendant de la réfraction de l'oeil opacifié.

3. Appareil selon les revendications 1 et 2 caractérisé en ce que la tête de ponçage (50) est pourvue d'une vis micrométrique (60) au moyen de laquelle la profondeur de ponçage du porte-outil de ponçage et de polissage (35) peut être réglée par rapport à l'oeil (10) en fonction de la réfraction de l'oeil opacifié ainsi que de l'écartement (A) existant entre la surface de référence (R1) et la surface de la cornée (OF).

4. Appareil selon la revendication 1 caractérisé en ce que la surface de ponçage (38) concave du porte-outil de ponçage ou de polissage (35) est pourvue d'une structure de surface cristallisée à grain fin ou de poussière de diamant.

5. Appareil selon les revendications 1 et 3 caractérisé en ce que :
   - le porte-outil (35) de ponçage ou de polissage est entraîné par un groupe d'entraînement à haute vitesse placé à l'intérieur de la tête de ponçage 50 ou externe, et
   - que la tête de ponçage (50) est placée sur une potence (150) connue en soi d'un support (200) conçu comme un pied, et
   - que la potence (150) avec la tête de ponçage (50) et le porte-outil de ponçage et de polissage (35) sont réglables par rapport à l'oeil dans le sens axial du centre optique (Z) en ce qui concerne la profondeur de ponçage qui dépend de la réfraction de l'oeil (10) opacifié.

FIG. 1

FIG. 2